# EUROPEAN PATENT APPLICATION

(11) **EP 3 816 177 A1**
(43) Date of publication of application: **05.05.2021**
(21) Application number: 19827103.3
(22) Date of filing: 27.06.2019
(51) Int. Cl.: C07K 1/22, C12M 1/00, C07K 1/34

(54) **METHOD FOR ISOLATING POLYPEPTIDE, METHOD FOR PRODUCING POLYPEPTIDE, AND APPARATUS FOR PURIFYING POLYPEPTIDE**

(30) Priority: 29.06.2018 JP 2018123981
(71) Applicant: Mitsubishi Chemical Corporation, Chiyoda-ku Tokyo 100-8251 (JP)
(72) Inventor: NOZAKI, Shinya, Tokyo 100-8251 (JP); TAKEHARA, Jun, Tokyo 100-8251 (JP); KURAMOTO, Ryoko, Tokyo 100-8251 (JP); OHARA, Shohei, Tokyo 100-8251 (JP); TASHIRO, Yoshiya, Tokyo 100-8251 (JP)
(74) Representative: Ter Meer Steinmeister & Partner
(86) International application number: PCT/JP2019/025701
(87) International publication number: WO 2020/004583

(57) **Abstract**

The present invention relates to a polypeptide separation method including a step (step A) of mixing the polypeptide, which is a monoclonal antibody, and a ligand in a liquid to form a complex of the polypeptide and the ligand and to obtain a liquid containing the complex, and a step (step B) of filtering the liquid containing the complex obtained in the step A.

## Description

### TECHNICAL FIELD

The present invention relates to a polypeptide separation method, a polypeptide production method, and a polypeptide purification device.

### BACKGROUND ART

A production of biopharmaceuticals based on bioactive substances, particularly organism-derived substances such as proteins, peptides and nucleic acids, requires a production and purification of molecules of the above substances on an actual process scale. Particularly, an increasing demand for a monoclonal antibody (mAb), which is a representative of the bioactive substances, has facilitated the development of cell culture techniques with high expression levels. As a result, there is an increasing demand for more efficient purification processes for the monoclonal antibody from a cell culture liquid.

As such a method for purifying a monoclonal antibody from a culture liquid, for example, Patent Literature 1 discloses a method using an affinity separating agent. In addition, Patent Literature 2 discloses a method using a filtration membrane.

### CITATION LIST

### PATENT LITERATURE

Patent Literature 1: WO 2015/199196
Patent Literature 2: JP-A-2009-221137

### SUMMARY OF INVENTION

### TECHNICAL PROBLEM

However, the purification method disclosed in Patent Literature 1 has a problem that an amount of a polypeptide adsorbed on the affinity separating agent is limited and the productivity of the purified polypeptide is poor. In addition, the productivity of the affinity separating agent is not high, energy is required for a production and immobilization of a carrier, the affinity separating agent should be stored under strict conditions in order to prevent a secession of the immobilized ligand, and the affinity separating agent *per se* has many problems.

Since the purification method disclosed in Patent Literature 2 is simple filtration, there is a problem that an impurity having a molecular weight close to that of a polypeptide to be purified remains. Further, in order to prevent such an impurity from remaining, there is a problem that strict filtration conditions should be set.

The present invention has been made in view of such circumstances, and an object of the present invention is to provide a high-efficiency polypeptide separation method applicable even on an industrial scale.

### SOLUTION TO PROBLEM

In the related art, although various methods such as a method using an affinity separating agent and a method using a filtration membrane have been studied as a method for obtaining a polypeptide from a culture liquid with high efficiency, a higher-efficiency polypeptide separation method is required. The present inventors have diligently made investigations and, as a result, have discovered a high-efficiency polypeptide separation method applicable even on an industrial scale.

Namely, the gist of the present invention is as follows.
[1] A polypeptide separation method including a step A and a step B as described below, wherein the polypeptide is a monoclonal antibody,
   step A: a step of mixing the polypeptide and a ligand in a liquid to form a complex of the polypeptide and the ligand and to obtain a liquid containing the complex, and
   step B: a step of filtering the liquid containing the complex obtained in the step A.
[2] The polypeptide separation method according to [1], wherein the complex is a complex of two or more molecules of the polypeptide with one molecule of the ligand.
[3] The polypeptide separation method according to [1] or [2], wherein in the step A, 2.1 mol or more of the polypeptide is mixed with 1 mol of the ligand.
[4] The polypeptide separation method according to any one of [1] to [3], wherein the ligand is protein A.
[5] The polypeptide separation method according to any one of [1] to [4], wherein a fractional molecular weight of a membrane for use in the filtration in step B is 10,000 to 250,000.
[6] The polypeptide separation method according to any one of [1] to [5], further including a step C as described below,
   step C: a step of further separating the complex separated in the step B into the polypeptide and the ligand.
[7] The polypeptide separation method according to [6], wherein in the step C, the separated complex is dissociated into the polypeptide and the ligand, and then further separated by filtration or liquid chromatography.
[8] The polypeptide separation method according to [6] or [7], wherein the further separation in the step C is performed by ion exchange chromatography.
[9] A polypeptide production method, including a purification step using the polypeptide separation method according to any one of [1] to [8].
[10] A polypeptide purification device, including a supply tank of a product to be purified, a diluent supply tank, a filter, and a detector.

### EFFECTS OF INVENTION

According to the polypeptide separation method of the present invention, filtration conditions can be relaxed, the method can be applied even on an industrial scale, and the productivity of the polypeptide is excellent.

In addition, according to the polypeptide production method of the present invention, filtration conditions can be relaxed, the method can be applied even on an industrial scale, and the productivity of the polypeptide is excellent.

Further, the polypeptide purification device according to the present invention is applicable for high-efficiency separation and production of the polypeptide even on an industrial scale.

### BRIEF DESCRIPTION OF DRAWINGS

[Fig. 1] Fig. 1 is a schematic diagram showing an embodiment of a polypeptide purification device according to the present invention.
[Fig. 2] Fig. 2 is a chromatogram of each liquid obtained in Reference Examples 1 to 9.
[Fig. 3] Fig. 3 is a chromatogram of each liquid obtained in Example 1.
[Fig. 4] Fig. 4 is a chromatogram of each liquid obtained in Comparative Example 1.
[Fig. 5] Fig. 5 is a chromatogram of each liquid obtained in Example 2.
[Fig. 6] Fig. 6 is a chromatogram of each liquid obtained in Comparative Example 2.
[Fig. 7] Fig. 7 is a chromatogram of each liquid obtained in Example 3.
[Fig. 8] Fig. 8 is a chromatogram of each liquid obtained in Example 4.
[Fig. 9] Fig. 9 is a chromatogram of each liquid obtained in Reference Example 10 and comparison targets.

### DESCRIPTION OF EMBODIMENTS

Hereinafter, although embodiments of the present invention are specifically described, it should not be construed that the present invention is limited to the following embodiments, and the present invention can be carried out by making various changes within the scope of a gist thereof. In the present description, the expression "to" is used as an expression including numerical values or physical property values before and after the expression. Additionally, in the present description, the expression "(meth)acrylic" means "acrylic", "methacrylic" or both, and the expression "(meth)acrylonitrile " means "acrylonitrile", "methacrylonitrile", or both.

### (Polypeptide Separation Method)

The polypeptide separation method according to the present invention includes a step A and a step B as described below, in which the polypeptide is a monoclonal antibody.
Step A: a step of mixing the polypeptide and a ligand in a liquid to form a complex of the polypeptide and the ligand and to obtain a liquid containing the complex.
Step B: a step of filtering the liquid containing the complex obtained in the step A.

It is preferable that the polypeptide separation method according to the present invention further includes a step C as described below.
Step C: a step of further separating the complex separated in the step B into the polypeptide and the ligand.

### (Step A)

The step A is a step of mixing a polypeptide and a ligand in a liquid to form a complex of the polypeptide and the ligand and to obtain a liquid containing the complex.

With the step A, it is possible to form a complex of the polypeptide and the ligand (hereinafter, which may be simply referred to as "the complex"), which is much larger than an impurity. The formation of the complex is preferred since the size difference from the impurity is larger and filtration conditions in the step B to be described later can be relaxed.

The monoclonal antibody has a small molecular weight and size distribution among polypeptides. Therefore, if there is a size difference between the monoclonal antibody and the impurity, the monoclonal antibody and the impurities can be separated by filtration. Therefore, the polypeptide separation method according to the present invention is extremely effective when the polypeptide is a monoclonal antibody.

When the liquid containing the complex is obtained, properties of the polypeptide and the ligand are not particularly limited, and they may be solids or liquids. Examples of the method of mixing the polypeptide and the ligand in a liquid include a method of adding a ligand to a liquid containing a polypeptide and mixing the two, a method of adding a polypeptide to a liquid containing a ligand and mixing the two, a method of mixing a liquid containing a polypeptide and a liquid containing a ligand, and a method of adding a polypeptide and a ligand to a liquid and mixing them. Among these methods, preferred are a method of adding a ligand to a liquid containing a polypeptide and mixing the two and a method of mixing a liquid containing a polypeptide and a liquid containing a ligand.

Examples of the liquid include water, a phosphate buffer solution, and an acetate buffer solution. Among these liquids, a phosphate buffer solution is preferable.

The liquid containing a polypeptide is not particularly limited as long as it contains a polypeptide, and a liquid containing a polypeptide and an impurity is preferred, and a cell culture liquid is more preferred, because the effect of the present invention is remarkably excellent.

In the present description, the term "impurity" refers to a by-product produced in the process of producing a polypeptide.

The mass average molecular weight of the polypeptide is preferably 10,000 or more, and is more preferably 20,000 or more, and is preferably 300,000 or less, and is more preferably 200,000 or less. When the mass average molecular weight of the polypeptide is 10,000 or more, a higher-order structure can be obtained and the functionality is excellent. When the mass average molecular weight of the polypeptide is 300,000 or less, the permeability to the affected part in the body is excellent.

In the present description, the mass average molecular weight of the polypeptide is a value measured by size exclusion chromatography. However, when it is difficult to measure the mass average molecular weight by size exclusion chromatography due to the type of the polypeptide or the like, values measured by electrophoresis using a fractionated gel or a method combining high performance liquid chromatography and a mass spectrometer may be used.

The mass average molecular weight of the impurity is preferably 10 or more, and is more preferably 1,000 or more, and is preferably 300,000 or less, and is more preferably 100,000 or less because excellent separability from the desired polypeptide can be obtained.

In the present description, the mass average molecular weight of the impurity is a value measured by size exclusion chromatography.

In the present description, the term "ligand" refers to a substance having an adsorption interaction with the polypeptide. The expression "having an adsorption interaction" means a state having an attractive force between molecules.

Examples of the ligand include: proteins such as protein A, protein G, protein L, Fc-binding protein, and avidin; peptides such as insulin; antibodies such as a monoclonal antibody; enzymes; hormones; DNA; RNA; and sugars such as heparin, Lewis X, and gangliosides. Among these ligands, proteins and peptides are preferred, proteins are more preferred, protein A, protein G, protein L and variants thereof are even more preferred, and protein A is particularly preferred, since excellent molecular recognition selectivity can be obtained.

The term "peptide" refers to a compound in which 10 to 50 amino acids are linked in a chain. The term "protein" refers to a compound in which 51 or more amino acids are linked in a chain, and may have a higher-order structure.

The mass average molecular weight of the ligand is preferably 10,000 or more, and is more preferably 20,000 or more, and is preferably 100,000 or less, and is more preferably 80,000 or less. When the mass average molecular weight of the ligand is 10,000 or more, the adsorptivity between the polypeptide and the ligand is excellent. When the mass average molecular weight of the ligand is 100,000 or less, the polypeptide and the ligand can be easily separated from each other and the characteristics of the ligand are excellent.

In the present description, the mass average molecular weight of the ligand is a value measured by size exclusion chromatography. However, when it is difficult to measure the mass average molecular weight by size exclusion chromatography due to the type of the ligand or the like, values measured by electrophoresis using a fractionated gel or a method combining high performance liquid chromatography and a mass spectrometer may be used.

Since the complex obtained by mixing the polypeptide and the ligand can make the size difference from the ligand larger and can greatly relax the filtration conditions (for example, the choice of the filtration membrane to be used can be increased, the number of filtrations can be reduced, or the like), it is preferable that two or more molecules of the polypeptide are allowed to form the complex with one molecule of the ligand. In addition, since the complex obtained by mixing the polypeptide and the ligand is easy to form, it is preferable that the amount of the polypeptide is 3 or less with respect to one molecule of the ligand.

A mixing ratio of the polypeptide and the ligand is preferably 2.1 mol or more, and is more preferably 2.5 mol or more, and is preferably 3.1 mol or less, and is more preferably 2.9 mol or less, of the polypeptide, with respect to 1 mol of the ligand. When the ratio of the polypeptide is 2.1 mol or more, a large amount of a complex in which two or more molecules of the polypeptide are allowed to form the complex with one molecule of the ligand is contained, the size difference from the ligand can be made larger, and the filtration conditions can be greatly relaxed. When the ratio of the polypeptide is 3.1 mol or less, the amount of the polypeptide which does not complex with the ligand can be reduced, and the amount of the polypeptide contained in the filtrate in the step B can be reduced.

The abundance ratio of the complex in which two or more molecules of the polypeptide are allowed to form the complex with one molecule of the ligand is preferably 70% or more, more preferably 90% or more, and still more preferably 95% or more, since the size difference from the ligand can be made larger, and the filtration conditions can be greatly relaxed.

The abundance ratio of the complex in which one molecule of the polypeptide is allowed to form the complex with one molecule of the ligand is preferably 30% or less, more preferably 10% or less, and still more preferably 5% or less, since the size difference from the ligand can be made larger, and the filtration conditions can be greatly relaxed.

The abundance of the polypeptide which is not allowed to form the complex with the ligand is preferably 15% or less, more preferably 10% or less, and still more preferably 5% or less.

In the present description, the abundance ratio of each of (i) the complex in which two or more molecules of the polypeptide are allowed to form the complex with one molecule of the ligand, (ii) the complex in which one molecule of the polypeptide is allowed to form the complex with one molecule of the ligand, and (iii) the polypeptide which is not allowed to form the complex with the ligand is taken as the area of the corresponding peak with respect to the total area of three types of peaks in the chromatogram when the liquid containing the complex obtained in the step (A) is measured by liquid chromatography. The three types of peaks are peaks caused by (i), (ii) and (iii).

The temperature at which the polypeptide and the ligand are mixed in a liquid to form a complex and to obtain a liquid containing the complex is preferably 5°C or higher, and is more preferably 10°C or higher, and is preferably 50°C or lower, and is more preferably 40°C or lower. When the temperature is 5°C or higher, the adsorptivity between the polypeptide and the ligand is excellent. When the temperature is 50°C or lower, the denaturation of the polypeptide or the ligand can be prevented, and the deterioration of the adsorptivity between the polypeptide and the ligand can be prevented.

A pH when the polypeptide and the ligand are mixed is preferably 3 or more, and is more preferably 4 or more, and is preferably 10 or less, and is more preferably 9 or less, since the polypeptide, which is a bioactive substance, has a stable structure.

### (Step B)

The step B is a step of filtering the liquid containing the complex obtained in the step A.

With the step B, the liquid containing the complex of the polypeptide and the ligand can be separated into the complex of the polypeptide and the ligand, and an impurity. The formation of the complex in the step A is preferred since the size difference between the complex and the impurity is larger, and as a result, the filtration conditions can be relaxed.

In the present description, the term "filtration" refers to separating molecules in a liquid through a filter medium. In filtration, the supernatant contains substances with larger sizes and the filtrate contains substances with smaller sizes.

Examples of the filter medium include a filtration membrane, a filter paper, a filter plate, a felt, and a mat. Among these filter media, a filtration membrane is preferable because excellent chemical stability and microfiltration characteristics can be obtained.

The filtration membrane (membrane for use in filtration) preferably has a fractional molecular weight of 10,000 or more, and more preferably 30,000 or more, because the pore size is not too small, many of the impurities can be contained in the filtrate, and the filtration efficiency is excellent. In addition, the filtration membrane preferably has a fractional molecular weight of 250,000 or less, and more preferably 200,000 or more, because the pore size is not too large and many of the complex can be contained in the supernatant.

In the present description, the fractional molecular weight of the filtration membrane refers to a molecular weight that can be retained by the filtration membrane at 90% or more. A value calculated from an inhibition rate of the filtration membrane with respect to a standard substance having five known molecular weights is used.

Examples of the material of the filtration membrane include a hydrophilic sulfone-based polymer membrane, a hydrophilic aromatic ether-based polymer membrane, a hydrophilic fluorine-based polymer membrane, a hydrophilic olefin-based polymer membrane, a cellulose-based membrane, a (meth)acrylic polymer membrane, a (meth)acrylonitrile-based polymer membrane, and a vinyl alcohol-based polymer membrane. Among these materials of the filtration membrane, because of having excellent hydrophilicity, a hydrophilic sulfone-based polymer membrane and a cellulose-based membrane are preferred, and a hydrophilic sulfone-based polymer membrane is more preferred.

The number of filtrations may be once or a plurality of times, and a plurality of times is preferred because excellent separability between the complex and the impurity can be obtained.

Examples of the filtration method include a method of applying a pressure perpendicularly to allow a filter medium to flow a liquid for filtration, such as a centrifugal method, and a tangential flow method. Among these filtration methods, the tangential flow method is preferred because the polypeptide can be handled stably and excellent filtration performance can be obtained even on an industrial scale.

### (Step C)

The step C is a step of further separating the complex separated in the step B into the polypeptide and the ligand.

The step C is preferred since the complex obtained by separation in the step B is further separated into the polypeptide and the ligand and a purified polypeptide can be obtained.

In the step C, it is preferable that the complex separated in the step B is dissociated into the polypeptide and the ligand, and then further separated into the polypeptide and the ligand, because the ligand can be recovered and reused and the environmental load can be reduced.

Examples of the method for dissociating the complex into the polypeptide and the ligand include adjustment of pH such as lowering pH, adjustment of the temperature such as raising the temperature, and adjustment of the salt concentration such as increasing the salt concentration. Among these dissociation methods, adjustment of pH and adjustment of the temperature are preferred, and adjustment of pH is more preferred because application is easy even on an industrial scale. When a monoclonal antibody is used as the polypeptide and protein A is used as the ligand, adjustment of pH is preferable as the dissociation method because application is easy even on an industrial scale.

Examples of the method for separating the polypeptide and the ligand after dissociation include filtration, liquid chromatography, and electrophoresis. Among these separation methods, filtration and liquid chromatography are preferred, and liquid chromatography is more preferred, because excellent separability and efficiency can be obtained.

Examples of the filter medium include a filtration membrane, a filter paper, a filter plate, a felt, and a mat. Among these filter media, a filtration membrane is preferable because excellent chemical stability and microfiltration characteristics can be obtained.

Examples of the material of the filtration membrane include a hydrophilic sulfone-based polymer membrane, a hydrophilic aromatic ether-based polymer membrane, a hydrophilic fluorine-based polymer membrane, a hydrophilic olefin-based polymer membrane, a cellulose-based membrane, a (meth)acrylic polymer membrane, a (meth)acrylonitrile-based polymer membrane, and a vinyl alcohol-based polymer membrane. Among these materials of the filtration membrane, because of having excellent hydrophilicity, a hydrophilic sulfone-based polymer membrane and a cellulose-based membrane are preferred, and a hydrophilic sulfone-based polymer membrane is more preferred.

The number of filtrations may be once or a plurality of times, and a plurality of times is preferred because excellent separability between the polypeptide and the ligand can be obtained.

Examples of the filtration method include a method of applying a pressure perpendicularly to allow a filter medium to flow a liquid for filtration, such as a centrifugal method, and a tangential flow method. Among these filtration methods, a tangential flow method is preferred because the polypeptide can be handled stably and excellent filtration performance can be obtained even on an industrial scale.

Examples of the mode of the liquid chromatography include affinity chromatography, ion exchange chromatography, reverse phase chromatography, and normal phase chromatography. Among these modes of the liquid chromatography, affinity chromatography and ion exchange chromatography are preferred because both the polypeptide and the ligand can be stably recovered, and ion exchange chromatography is more preferred because excellent efficiency can be obtained.

### (Polypeptide Production Method)

The polypeptide production method according to the present invention includes a purification step using the polypeptide separation method according to the present invention.

The polypeptide production method preferably includes a biological culture or synthesis step and a formulation step, in addition to the purification step using the above polypeptide separation method, from the viewpoint of industrial production.

The biological culture is a step of artificially culturing cells or the like to produce a polypeptide.

The synthesis step is a step of producing a polypeptide by using an amino acid or a short-chain polypeptide as a raw material and using an organic synthesis reaction.

The biological culture or synthesis step is not particularly limited, and a known biological culture or synthesis step can be used.

The formulation step is a step of blending components necessary for the polypeptide to be purified and molding.

The formulation step is not particularly limited, and a known formulation step can be used.

The purification step using the above polypeptide separation method is preferably performed using the following purification device because energy can be saved and excellent efficiency can be obtained.

### (Polypeptide Purification Device)

The polypeptide purification device according to the present invention includes a supply tank of a product to be purified, a diluent supply tank, a filter, and a detector.

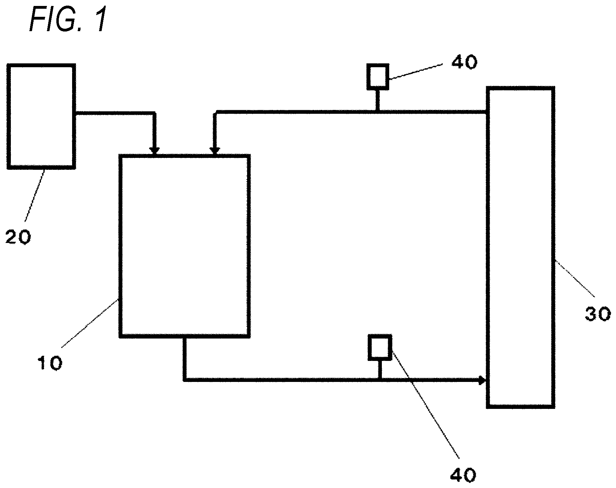

Fig. 1 is a schematic diagram showing an embodiment of the polypeptide purification device according to the present invention. Hereinafter, although the polypeptide purification device will be described with reference to the drawings, the present invention is not limited to the drawings.

The polypeptide purification device shown in Fig. 1 includes a supply tank 10 of a product to be purified, a diluent supply tank 20, a filter 30, and detectors 40. The diluent supply tank 20 is connected to the supply tank 10 of a product to be purified such that a diluent can be supplied thereto. The supply tank 10 of a product to be purified and the filter 30 are connected to each other so as to perform circulation and purification therebetween for a plurality of times. The detectors 40 are separately connected between from the supply tank 10 of a product to be purified to the filter 30 and between from the filter 30 to the supply tank 10 of a product to be purified.

The supply tank 10 of a product to be purified is a tank for supplying a product to be purified to the filter 30, and stores a polypeptide and a ligand. The polypeptide and the ligand are stored in the supply tank 10 of a product to be purified in a liquid state in which a complex is formed, and when the polypeptide and the ligand are passed through the filter 30, the complex (a supernatant) of the polypeptide and the ligand and the impurity (a filtrate) can be separated from each other. Since the separability between the complex and the impurity is excellent, it is preferable to circulate and pass the product to be purified through the filter 30 a plurality of times.

In order to stably supply the product to be purified from the supply tank 10 of a product to be purified to the filter 30, it is preferable to connect a supply pump near an outlet of the supply tank 10 of a product to be purified.

The material of the supply tank 10 of a product to be purified is preferably a hydrophilic material, and more preferably a resin having a hydrophilic surface, because nonspecific adsorption with the polypeptide or the ligand is not caused.

The diluent supply tank 20 is a tank for supplying the diluent to the supply tank 10 of a product to be purified.

When the product to be purified is passed through the filter 30, the product to be purified is concentrated, and the filtration efficiency gradually decreases. However, when a diluent is supplied from the diluent supply tank 20, a decrease in filtration efficiency can be prevented.

The degree of dilution may be appropriately set, and it is preferable to supply the diluent such that the concentration of the complex in the liquid is constant.

The concentration of the complex in the liquid is preferably 1 g/L or more, and is more preferably 5 g/L or more, and is preferably 300 g/L or less, and is more preferably 200 g/L or less. When the concentration of the complex in the liquid is 1 g/L or more, the separation can be completed in a short time. When the concentration of the complex in the liquid is 300 g/L or less, the precipitation of the complex can be prevented.

The filter 30 is for purifying the product to be purified, namely, for separating the complex and the impurity.

Examples of the filter medium include a filtration membrane, a filter paper, a filter plate, a felt, and a mat. Among these filter media, a filtration membrane is preferable because excellent chemical stability and microfiltration characteristics can be obtained.

Examples of the material of the filtration membrane include a hydrophilic sulfone-based polymer membrane, a hydrophilic aromatic ether-based polymer membrane, a hydrophilic fluorine-based polymer membrane, a hydrophilic olefin-based polymer membrane, a cellulose-based membrane, a (meth)acrylic polymer membrane, a (meth)acrylonitrile-based polymer membrane, and a vinyl alcohol-based polymer membrane. Among these materials of the filtration membrane, because of having excellent hydrophilicity, a hydrophilic sulfone-based polymer membrane and a cellulose-based membrane are preferred, and a hydrophilic sulfone-based polymer membrane is more preferred.

The detectors 40 are preferably installed in order to keep the system of the purification device stable.

Examples of the detector 40 include a pressure detector, a concentration detector, a mass detector, a flow rate detector, and a thermometer. Among these detectors 40, it is preferable to use a flow rate detector and a pressure detector in combination because the stability in the system of the purification device having a filtration membrane with low pressure durability is excellent.

### (Applications)

The polypeptide separation method according to the present invention and the polypeptide production method according to the present invention can be applied even on an industrial scale, and the productivity of the polypeptide is excellent.

In addition, the polypeptide purification device according to the present invention is applicable for high-efficiency separation and production of the polypeptide even on an industrial scale.

The polypeptide obtained by the separation method according to the present invention, the polypeptide obtained by the production method according to the present invention, and the polypeptide obtained by using the purification device according to the present invention can be suitably used, for example, for medicines for medical treatment, functional foods, intermediates for synthesizing high value-added compounds, and the like, and can be particularly preferably used for medicines for medical treatment because of being excellent in improving the health condition.

### Examples

Hereinafter, although the present invention is explained further more concretely by ways of Examples, the present invention is not limited to following Examples, unless the gist of the present invention is exceeded.

### [Reference Examples 1 to 9]

To a 2 mL polypropylene tube, a 10 g/L monoclonal antibody aqueous solution, a 50 g/L protein A aqueous solution derived from *Staphylococcus aureus,* and a phosphate buffer solution (0.0027 mol/L potassium chloride, 0.137 mol/L sodium chloride, and 0.01 mol/L phosphoric acid, pH: 7.4) were added with the blending amount shown in Table 1, followed by stirring, and the mixture was allowed to stand at 20°C for 2 hours to obtain a liquid containing a complex of a polypeptide and a ligand.

The fractionation status of the components in the obtained liquid containing the complex of the polypeptide and the ligand was confirmed by size exclusion chromatography. The conditions for the size exclusion chromatography were as described below.
Column: "TSK Gel G3000 SWXL" (product name, inner diameter: 8 mm, length: 300 mm)
Mobile phase: phosphate buffer solution (0.0027 mol/L potassium chloride, 0.137 mol/L sodium chloride, and 0.01 mol/L phosphoric acid, pH: 7.4)
Flow velocity: 1.0 mL/min
Detection wavelength: 280 nm
Sample volume: 0.01 mL

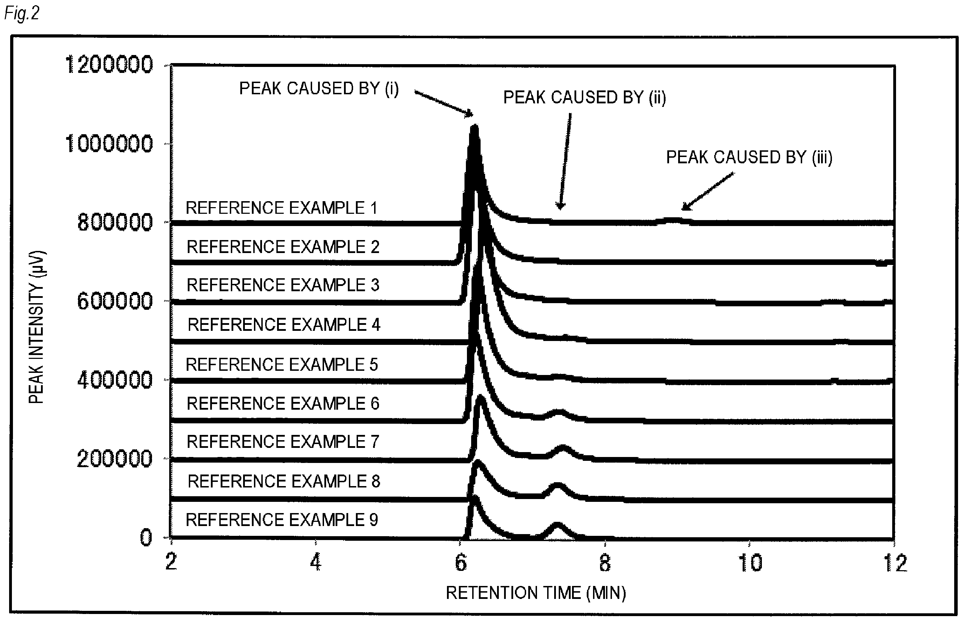

The obtained chromatograms are collectively shown in Fig. 2.

From the peak position of the standard substance, it was confirmed that the peak existing at the retention time of about 6 minutes was the peak caused by (i) described below, the peak existing at the retention time of about 7 minutes was the peak caused by (ii) described below, and the peak existing at the retention time of about 9 minutes was the peak caused by (iii) described below.

In addition, from the obtained chromatograms, an abundance ratio of each complex or polypeptide was calculated from the peaks caused by the following (i) to (iii).
(i) a complex in which two or more molecules of a polypeptide are allowed to form the complex with one molecule of a ligand
(ii) a complex in which one molecule of a polypeptide is allowed to form the complex with one molecule of a ligand
(iii) a polypeptide which does not form a complex with a ligand

The results are shown in Table 2.

**[Table 1]**

| | Blending amount | | | Molar ratio | | Mass ratio | |
|---|---|---|---|---|---|---|---|
| | Polypeptide aqueous solution (µL) | Ligand aqueous solution (µL) | Phosphate buffer solution (µL) | Polypeptide | Ligand | Polypeptide | Ligand |
| Reference Example 1 | 900 | 20 | 80 | 3.0 | 1 | 9.0 | 1 |
| Reference Example 2 | 840 | 20 | 140 | 2.8 | 1 | 8.4 | 1 |
| Reference Example 3 | 780 | 20 | 200 | 2.6 | 1 | 7.8 | 1 |
| Reference Example 4 | 720 | 20 | 260 | 2.4 | 1 | 7.2 | 1 |
| Reference Example 5 | 660 | 20 | 320 | 2.2 | 1 | 6.6 | 1 |
| Reference Example 6 | 600 | 20 | 380 | 2.0 | 1 | 6.0 | 1 |
| Reference Example 7 | 540 | 20 | 440 | 1.8 | 1 | 5.4 | 1 |
| Reference Example 8 | 480 | 20 | 500 | 1.6 | 1 | 4.8 | 1 |
| Reference Example 9 | 400 | 20 | 580 | 1.3 | 1 | 4.0 | 1 |

**[Table 2]**

| | Abundance Ratio (%) | | |
|---|---|---|---|
| | (i) | (ii) | (iii) |
| Reference Example 1 | 89.7 | 0.0 | 10.3 |
| Reference Example 2 | 99.6 | 0.0 | 0.39 |
| Reference Example 3 | 100.0 | 0.0 | 0.0 |
| Reference Example 4 | 94.8 | 5.2 | 0.0 |
| Reference Example 5 | 92.8 | 7.2 | 0.0 |
| Reference Example 6 | 86.3 | 13.7 | 0.0 |
| Reference Example 7 | 78.6 | 21.4 | 0.0 |
| Reference Example 8 | 69.1 | 30.9 | 0.0 |
| Reference Example 9 | 67.9 | 32.1 | 0.0 |

### [Example 1]

To a 2 mL polypropylene tube, a 10 g/L monoclonal antibody aqueous solution, a 50 g/L protein A aqueous solution derived from *Staphylococcus aureus,* and a phosphate buffer solution (0.0027 mol/L potassium chloride, 0.137 mol/L sodium chloride, and 0.01 mol/L phosphoric acid, pH: 7.4) were added with the blending amount of Reference Example 9 shown in Table 1, followed by stirring, and the mixture was allowed to stand at 20°C for 2 hours to obtain a liquid containing a complex of a polypeptide and a ligand.

The obtained liquid containing the complex was injected into a membrane filtration device "Amicon Ultra-0.5" (product name, manufactured by Merck KGaA, Ultracel, fractional molecular weight: 100,000) and centrifuged according to the following procedures (1) to (9) to obtain a supernatant and a filtrate. The conditions for centrifugation were 15,000 (×g) for 3 minutes.
(1) Filtration is performed by centrifugation to obtain a supernatant and a filtrate. The entire filtrate is collected from the membrane filtration device and stored separately.
(2) A phosphate buffer solution is added to the supernatant obtained in (1).
(3) Filtration is performed by centrifugation to obtain a supernatant and a filtrate. The entire filtrate is collected from the membrane filtration device and stored separately.
(4) A phosphate buffer solution is added to the supernatant obtained in (3).
(5) Filtration is performed by centrifugation to obtain a supernatant and a filtrate. The entire filtrate is collected from the membrane filtration device and stored separately.
(6) A phosphate buffer solution is added to the supernatant obtained in (5).
(7) Filtration is performed by centrifugation to obtain a supernatant and a filtrate. The entire filtrate is collected from the membrane filtration device and stored separately.
(8) A phosphate buffer solution is added to the supernatant obtained in (7).
(9) Filtration is performed by centrifugation to obtain a supernatant and a filtrate. The entire filtrate is collected from the membrane filtration device and stored separately.

Regarding the liquid before the filtration in the above (1), the supernatant obtained in the above (9), the filtrate obtained in the above (1), the filtrate obtained in the above (5), and the filtrate obtained in the above (9), the fractionation status of the components in each liquid was confirmed by size exclusion chromatography. The conditions for the size exclusion chromatography were the same as those in Reference Example 9.

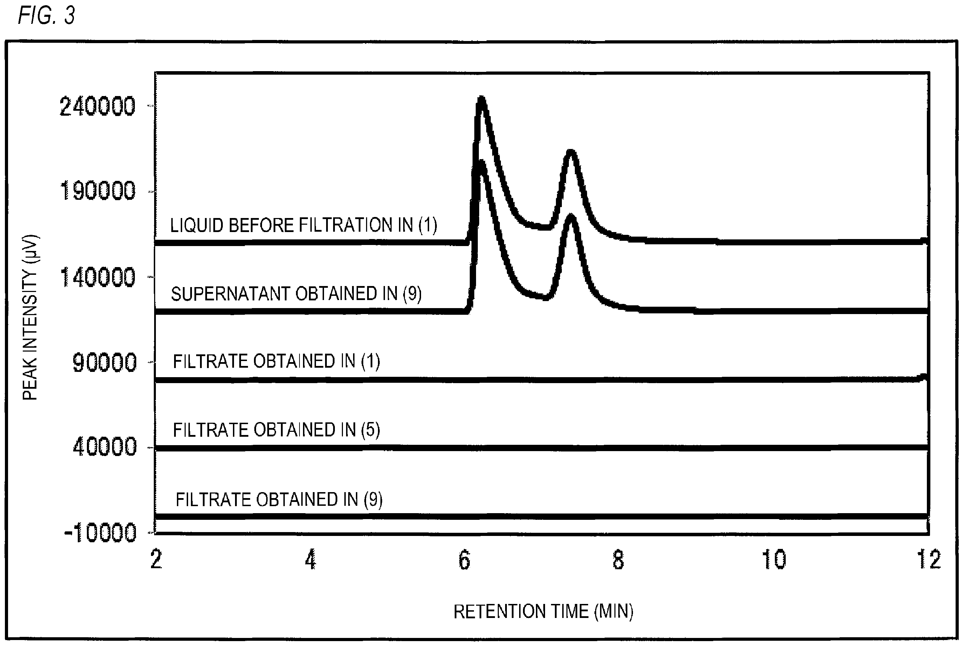

The obtained chromatograms are collectively shown in Fig. 3.

### [Comparative Example 1]

The fractionation status of the components in each liquid was confirmed by the operation same as in Example 1, except that the 50 g/L protein A aqueous solution derived from *Staphylococcus aureus* was not added.

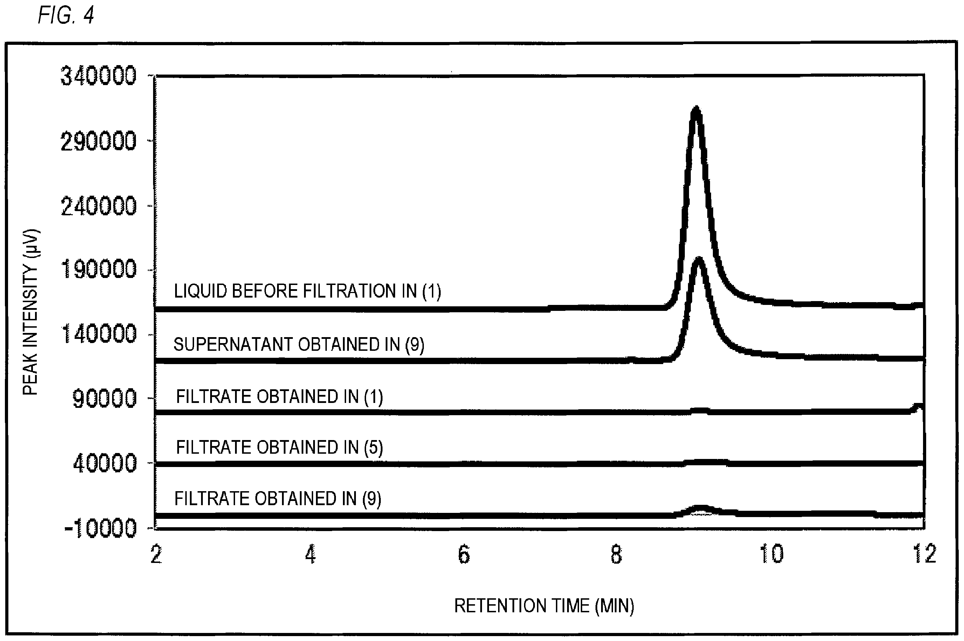

The obtained chromatograms are collectively shown in Fig. 4.

From the chromatograms obtained in Example 1, it was confirmed that many of the complex in which two or more molecules of the polypeptide were allowed to form the complex with one molecule of the ligand and the complex in which one molecule of the polypeptide was allowed to form the complex with one molecule of the ligand could remain in the supernatant without passing through the filtration membrane. It was also confirmed that many of the complex could remain in the supernatant without passing through the filtration membrane, even with a plurality of filtrations to remove impurities.

On the other hand, from the chromatograms obtained in Comparative Example 1, it was confirmed that a part of the polypeptide which was not allowed to form the complex with the ligand passed through the filtration membrane.

### [Example 2]

The fractionation status of the components in each liquid was confirmed by the operation same as in Example 1, except that the membrane filtration device was change from "Amicon Ultra-0.5" to "Apollo 7ml" (product name, manufactured by Orbital Biosciences, fractional molecular weight: 150,000).

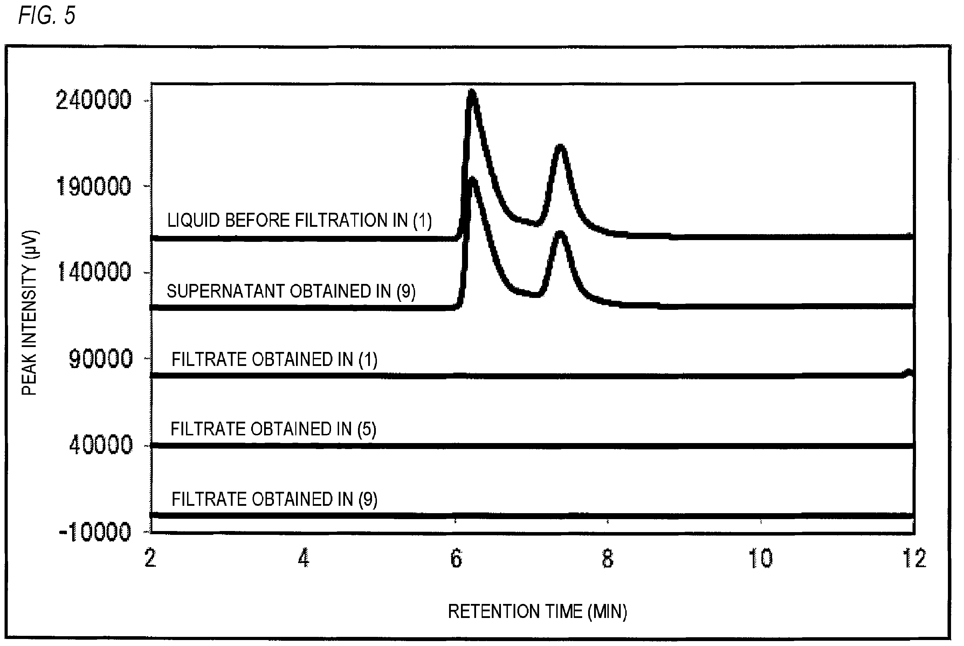

The obtained chromatograms are collectively shown in Fig. 5.

### [Comparative Example 2]

The fractionation status of the components in each liquid was confirmed by the operation same as in Example 2, except that the 50 g/L protein A aqueous solution derived from *Staphylococcus aureus* was not added.

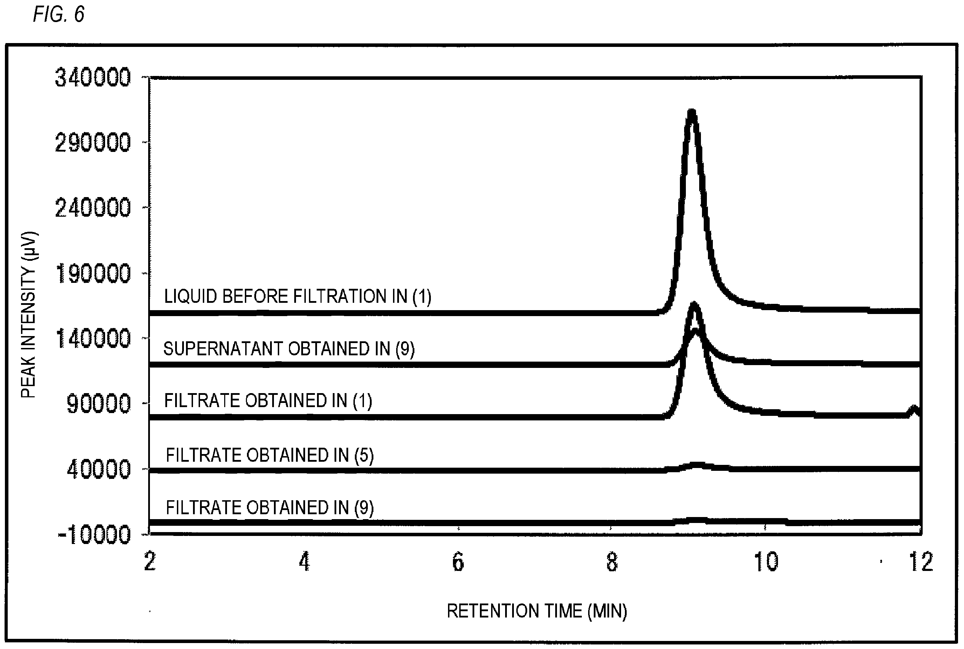

The obtained chromatograms are collectively shown in Fig. 6.

From the chromatograms obtained in Example 2, it was confirmed that many of the complex in which two or more molecules of the polypeptide were allowed to form the complex with one molecule of the ligand and the complex in which one molecule of the polypeptide was allowed to form the complex with one molecule of the ligand could remain in the supernatant without passing through the filtration membrane. It was also confirmed that many of the complex could remain in the supernatant without passing through the filtration membrane, even with a plurality of filtrations to remove impurities.

On the other hand, from the chromatograms obtained in Comparative Example 2, it was confirmed that many of the polypeptide which was not allowed to form the complex with the ligand passed through the filtration membrane.

### [Example 3]

The fractionation status of the components in each liquid was confirmed by the operation same as in Example 1, except that the blending amount of Reference Example 9 was changed to the blending amount of Reference Example 3 in Table 1.

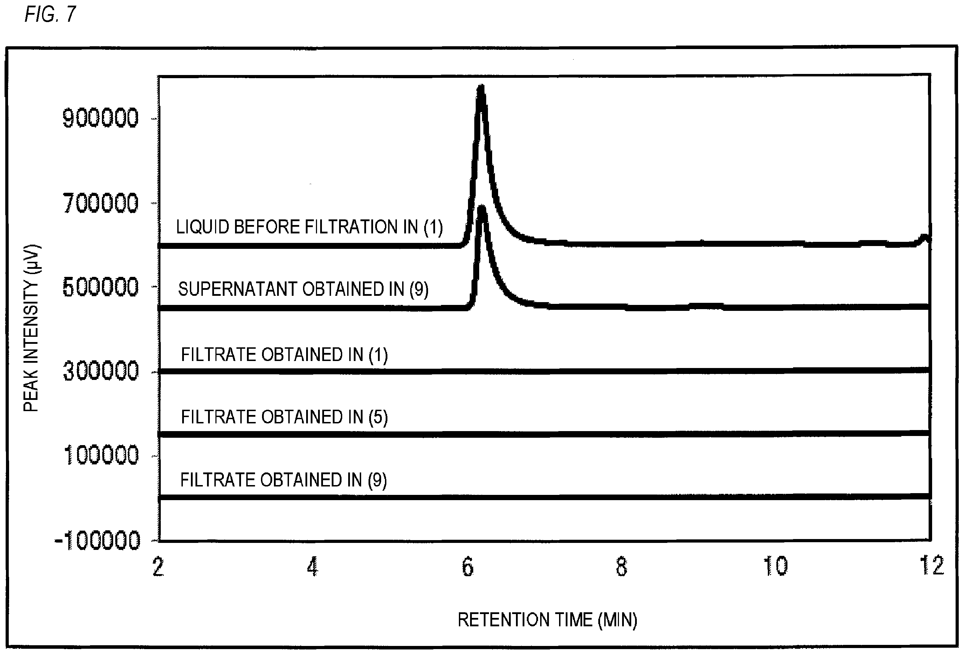

The obtained chromatograms are collectively shown in Fig. 7.

From the chromatograms obtained in Example 3, it was confirmed that many of the complex in which two or more molecules of the polypeptide were allowed to form the complex with one molecule of the ligand could remain in the supernatant without passing through the filtration membrane. It was also confirmed that many of the complex could remain in the supernatant without passing through the filtration membrane, even with a plurality of filtrations to remove impurities.

On the other hand, from the chromatograms obtained in Comparative Example 1, it was confirmed that a part of the polypeptide which was not allowed to form the complex with the ligand passed through the filtration membrane.

### [Example 4]

The fractionation status of the components in each liquid was confirmed by the operation same as in Example 2, except that the blending amount of Reference Example 9 was changed to the blending amount of Reference Example 3 in Table 1.

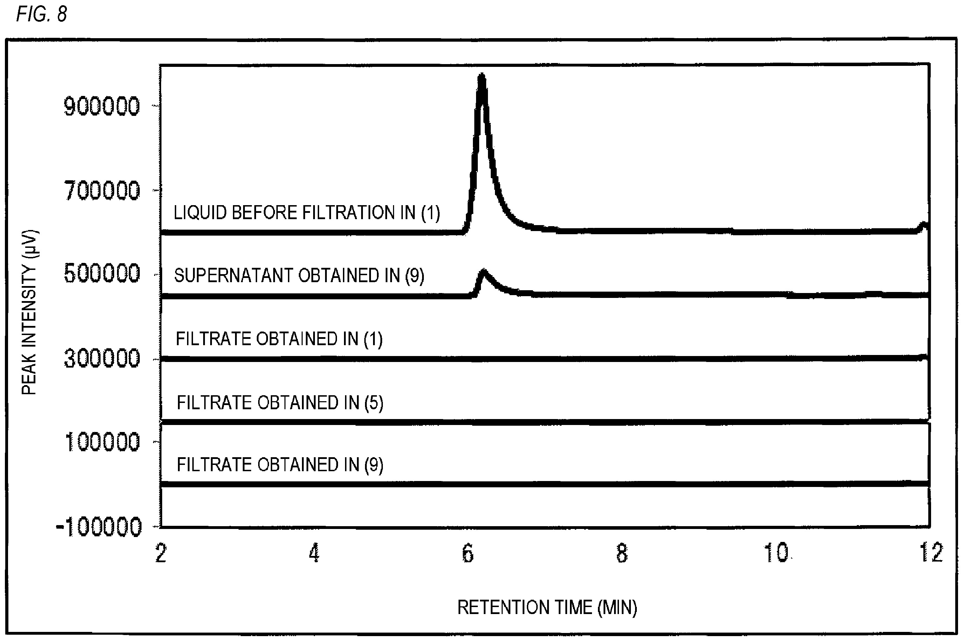

The obtained chromatograms are collectively shown in Fig. 8.

From the chromatograms obtained in Example 4, it was confirmed that many of the complex in which two or more molecules of the polypeptide were allowed to form the complex with one molecule of the ligand could remain in the supernatant without passing through the filtration membrane. It was also confirmed that many of the complex could remain in the supernatant without passing through the filtration membrane, even with a plurality of filtrations to remove impurities.

On the other hand, from the chromatograms obtained in Comparative Example 2, it was confirmed that many of the polypeptide which was not allowed to form the complex with the ligand passed through the filtration membrane.

### [Reference Example 10]

In a 2 mL of polypropylene tube, 200 µL of a 10 g/L monoclonal antibody aqueous solution, 200 µL of a 50 g/L protein A aqueous solution derived from *Staphylococcus aureus,* and 100 µL of a phosphate buffer solution (0.0027 mol/L potassium chloride, 0.137 mol/L sodium chloride, and 0.01 mol/L phosphoric acid, pH: 7.4) were stirred, to obtain a liquid containing a complex of a polypeptide and a ligand. The obtained liquid was allowed to stand at 20°C for 2 hours, and then the liquid after standing was adjusted to have a pH of 4.5 with a 1.0 mol/L acetic acid aqueous solution.

As comparison targets, a liquid containing a polypeptide obtained by mixing 200 µL of a monoclonal antibody aqueous solution with 300 µL of a phosphate buffer solution and a liquid containing a ligand obtained by mixing 200 µL of a protein A aqueous solution with 300 µL of a phosphate buffer solution were prepared.

The fractionation status of the three prepared liquids was confirmed by cation exchange chromatography. The conditions for the cation exchange chromatography were as described below.
Column: "ChromSpeed S103" (product name, manufactured by Mitsubishi Chemical Corporation, inner diameter: 5 mm, length: 100 mm)
Mobile phase: 20 mmol/L sodium acetate aqueous solution (pH: 4.5)
Flow velocity: 1.0 mL/min
Detection wavelength: 280 nm
Sample volume: 0.01 mL

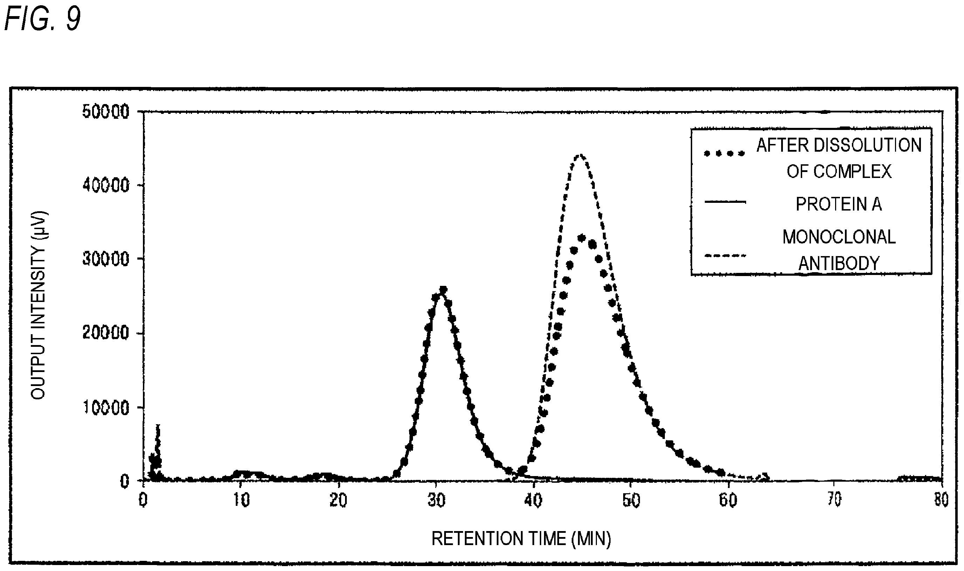

The obtained chromatograms are collectively shown in Fig. 9.

It was confirmed from Fig. 9 that when the pH was adjusted as in Reference Example 10, the complex of the polypeptide (a monoclonal antibody) and the ligand (a protein A) could be dissociated into the polypeptide and the ligand, and the subsequent cation exchange chromatography can separate the polypeptide and the ligand.

From the results of these Examples, Comparative Examples, and Reference Examples, it was confirmed that application on an industrial scale was possible without using an affinity separating agent requiring complicated regeneration processing, and the polypeptide could be recovered with high purity and the ligand could also be recovered with high purity under mild pH conditions of pH 4 or more which does not damage antibodies.

Although the present invention has been described in detail with reference to specific embodiments, it will be apparent to those skilled in the art that various changes and modifications can be made without departing from the spirit and scope of the invention. The present application is based on a Japanese Patent Application (Japanese Patent Application No. 2018-123981) filed on June 29, 2018, contents of which are incorporated herein by reference.

### INDUSTRIAL APPLICABILITY

The polypeptide separation method according to the present invention and the polypeptide production method according to the present invention can be applied even on an industrial scale, and the productivity of the polypeptide is excellent. In addition, the polypeptide purification device according to the present invention is applicable for high-efficiency separation and production of the polypeptide even on an industrial scale.

The polypeptide obtained by the separation method according to the present invention, the polypeptide obtained by the production method according to the present invention, and the polypeptide obtained by using the purification device according to the present invention can be suitably used, for example, for medicines for medical treatment, functional foods, intermediates for synthesizing high value-added compounds, and the like, and can be particularly preferably used for medicines for medical treatment because of being excellent in improving the health condition.

### REFERENCE SIGNS LIST

10 supply tank of a product to be purified
20 diluent supply tank
30 filter
40 detector

## Claims

1. A polypeptide separation method comprising a step A and a step B as described below, wherein the polypeptide is a monoclonal antibody,
step A: a step of mixing the polypeptide and a ligand in a liquid to form a complex of the polypeptide and the ligand and to obtain a liquid containing the complex, and
step B: a step of filtering the liquid containing the complex obtained in the step A.

2. The polypeptide separation method according to claim 1, wherein the complex is a complex of two or more molecules of the polypeptide with one molecule of the ligand.

3. The polypeptide separation method according to claim 1 or 2, wherein in the step A, 2.1 mol or more of the polypeptide is mixed with 1 mol of the ligand.

4. The polypeptide separation method according to any one of claims 1 to 3, wherein the ligand is protein A.

5. The polypeptide separation method according to any one of claims 1 to 4, wherein a fractional molecular weight of a membrane for use in the filtration in step B is 10,000 to 250,000.

6. The polypeptide separation method according to any one of claims 1 to 5, further comprising a step C as described below,
step C: a step of further separating the complex separated in the step B into the polypeptide and the ligand.

7. The polypeptide separation method according to claim 6, wherein in the step C, the separated complex is dissociated into the polypeptide and the ligand, and then further separated by filtration or liquid chromatography.

8. The polypeptide separation method according to claim 6 or 7, wherein the further separation in the step C is performed by ion exchange chromatography.

9. A polypeptide production method, comprising a purification step using the polypeptide separation method according to any one of claims 1 to 8.

10. A polypeptide purification device, comprising a supply tank of a product to be purified, a diluent supply tank, a filter, and a detector.
